Europäisches Patentamt

European Patent Office

Office européen des brevets

(11) Veröffentlichungsnummer: **0 399 324**
**A2**

## (12) EUROPÄISCHE PATENTANMELDUNG

(21) Anmeldenummer: **90109040.7**

(22) Anmeldetag: **14.05.90**

(51) Int. Cl.⁵: **A61B 6/00, G01N 23/04**

(30) Priorität: **23.05.89 DE 8906386 U**

(43) Veröffentlichungstag der Anmeldung:
**28.11.90 Patentblatt 90/48**

(84) Benannte Vertragsstaaten:
**AT BE CH DE ES FR IT LI NL**

(71) Anmelder: **PICKER INTERNATIONAL GMBH**
**Bärmannstrasse 38**
**D-8000 München 60(DE)**

(72) Erfinder: **Budde, Hans**
**Wirtsheide 8c**
**D-4980 Bünde(DE)**
Erfinder: **Jerolm, Hermann**
**Fiestelerweg 16**
**D-4515 Bad Essen(DE)**
Erfinder: **Petersmann, Helmut**
**Kronsbrink 11**
**D-4515 Bad Essen(DE)**
Erfinder: **Sohns, Jürgen**
**Friedhofstrasse 2**
**D-4980 Bünde(DE)**
Erfinder: **Schneider, Eli-Josef**
**Wollriede 2**
**D-4992 Espelkamp(DE)**
Erfinder: **Wehbrink, Herbert**
**Wehe 61**
**D-4993 Rahden(DE)**
Erfinder: **Paxmann, Peter**
**Krähenstrasse 17**
**D-8000 München 60(DE)**

(74) Vertreter: **Patentanwälte Viering & Jentschura**
**Steinsdorfstrasse 6**
**D-8000 München 22(DE)**

(54) **Angiographie-Gerät.**

(57) Angiographie-Gerät mit einer Strahlereinheit (7) und einer Bildverstärkereinheit (8), die im Bereich der Enden eines C-Bogens (4) angebracht sind, welcher um seinen Krümmungsmittelpunkt drehverstellbar an einem L-Arm (2) befestigt ist, der an einer horizontalen Welle (3) schwenkverstellbar an der Vorderseite eines deckenfreien Standes (1) angebracht ist. Um eine leicht Verstellbarkeit und eine hohe Standfestigkeit zu erreichen, ist die horizontale Schwenkwelle (3) des L-Arms (2) höhenverstellbar und mit der Schwenkwelle (3) ist ein mit dieser gegensinnig verstellbares, an der Rückseite des Standes (1) angeordnetes Gegengewicht (9) gekuppelt.

Fig.1

## Angiographie-Gerät

Die Erfindung betrifft ein Angiographie-Gerät mit einer Strahlereinheit und einer Bildverstärkereinheit, die im Bereich der Enden eines C-Bogens angebracht sind, welcher um seinen Krümmungsmittelpunkt drehverstellbar an einem L-Arm befestigt ist, der an einer horizontalen Welle schwenkverstellbar an der Vorderseite eines deckenfreien Standes angebracht ist.

Bei derartigen Angiographie-Geräten kann durch ein Verschwenken des L-Armes und/oder ein Drehen des C-Bogens um dessen Krümmungsmittelpunkt die Strahlungsrichtung der Strahlereinheit in breitem Maße geändert werden, um optimale Aufnahmen von den zu betrachtenden Körperteilen des auf einem Tisch liegenden Patienten zu erhalten. Jedoch muß der Stand, wenn er deckenfrei sein soll, d.i. nur am Zimmerboden, nicht aber auch an der Zimmerdecke befestigt ist, derart festgelegt werden, daß das Kippmoment zuverlässig kompensiert wird, welches unter dem Gewicht des einseitig auskragenden C-Bogens und der daran sitzenden Bildverstärkereinheit und Strahlereinheit auf den Stand einwirkt.

Durch die Erfindung wird die Aufgabe gelöst, ein Angiographie-Gerät eingangs erwähnter Art zu schaffen und so auszubilden, daß dessen Einsatzmöglichkeiten bei leichter Verstellbarkeit und hoher Standfestigkeit des Gerätes erweitert werden können.

Dies wird erfindungsgemäß dadurch erreicht, daß die horizontale Schwenkwelle des L-Arms höhenverstellbar ist und daß mit der Schwenkwelle ein mit dieser gegensinnig verstellbares, an der Rückseite des Standes angeordnetes Gegengewicht gekuppelt ist.

Mit Hilfe des Gegengewichtes gelingt es, das Kippmoment aus dem Gewicht des C-Bogens und der daran sitzenden Einheiten weitgehend zu kompensieren, wodurch eine problemlose Abstützung des Standes am Zimmerboden bei hoher Standfestigkeit des Gerätes verwirklicht werden kann. Darüberhinaus wird durch das Gegengewicht eine verhältnismäßig leichtgängige Höhenverstellbarkeit des C-Bogens erreicht, so daß der Antrieb für diese Verstellbewegung entlastet ist und daher auf eine geringere Antriebsleistung ausgelegt sein kann.

Aufgrund des Vorhandenseins des Gegengewichtes gibt es auch die Möglichkeit, den Stand für zusätzliche Verstellmöglichkeiten des Gerätes und dessen platzsparendes Verfahren in eine Parkstellung um eine vertikale Achse drehbar zu gestalten, die durch den Geräteschwerpunkt oder nahe an diesem verläuft. Zwar kann sich der aktuelle Geräteschwerpunkt bei einer Drehung des C-Bogens um dessen Krümmungsmittelpunkt ändern, jedoch verläuft auch dabei die vertikale Achse aufgrund des Vorhandenseins des Gegengewichtes noch im Bereich des Geräteschwerpunktes, wodurch es nicht zu konstruktiven Abstützschwierigkeiten bei der Verwirklichung der Drehbarkeit des Standes kommt.

Die Erfindung bietet darüberhinaus die problemfreie Möglichkeit, den Stand auf Schienen zu setzen und für eine zusätzliche Erweiterung der Verstellmöglichkeiten seitlich verfahrbar zu machen, weil es durch das Vorhandensein des Gegengewichtes nicht erforderlich ist, den Stand am Zimmerboden zu verankern.

Ferner wird die Stabilität des Gerätes weiter verbessert, wenn der an der horizontalen Schwenkwelle befestigte Schenkel des L-Armes unter Ausbildung eines zweiten Gegengewichtes verlängert ist, wodurch eine wesentliche Kompensation auch des seitlichen Kippmomentes erreicht werden kann, das aufgrund des über den einseitig auskragenden L-Arm einwirkenden Gewichtes des C-Bogens und der von diesem getragenen Strahler- und Bildverstärkereinheiten einwirkt. Durch die Ausbildung dieses zweiten Gegengewichtes wird außerdem die Antriebskraft für das Schwenken des L-Arms um die horizontale Achse verringert, so daß auch dieser Antrieb auf eine geringere Antriebsleistung ausgelegt sein kann.

Vorzugsweise sind die Strahlereinheit und die Bildverstärkereinheit an der der Schwenkwelle zugewandten Seite des C-Bogens gegenüber diesem derart versetzt angeordnet, daß die Achse der horizontalen Welle und die Strahlachse einander schneiden. Hierdurch ist der Zugang zu dem Patienten verbessert und ist das Gerät gut geeignet für einen Bi-Planebetrieb in der Kombination mit einem Obertischbildverstärker und einer Untertischstrahlereinheit. Hierbei wird der C-Bogen auf die Lateralaufnahmerichtung eingestellt, wohingegen der Obertischbildverstärker für die vertikale Aufnahmerichtung eingesetzt wird. Durch einen derartigen Betrieb wird die Strahlenbelastung für das Bedienpersonal auf ein Minimum reduziert.

Zu dem erfindungsgemäßen Gerät kann ein fahrbares Bedienpult gehören, welches über ein Kabel angeschlossen ist. Um möglichst wenige Steueradern zwischen dem Bedienpult und der Steuereinheit zu haben, können die Daten seriellbidirektional übertragen und in der Steuereinheit sowie dem Bedienpult verarbeitet werden. Der bidirektionale Datenfluß erlaubt die Anzeige der Systemparameter am Bedienpult.

Vorzugsweise weist das erfindungsgemäße Angiographie-Gerät einen Aufnahmepositionsspeicher auf, der von dem fahrbaren Bedienpult her, welches Aufnahmepositionstasten enthält, abrufbar ist. In dieser Weise können immer wiederkehrende Aufnahmepositionen gespeichert und durch Betätigen der jeweils zugeordneten Taste selbsttätig angefahren werden. Bevorzugt weist hierzu das Gerät eine speicherprogrammierbare Steuerung (SPS) auf, von welcher die Geräteantriebe und auch andere Gerätefunktionen gesteuert werden.

Bevorzugt ist außerdem das Gerät in seinen Gerätebewegungen über einen Infrarotsender fernbedienbar ausgeführt und weist hierzu einen Infrarotfernbedienungseingang zur selektiven Ansteuerung des jeweiligen Antriebes der Geräteteile auf.

Die Erfindung wird im folgenden anhand einer Ausführungsform erläutert, die wenigstens schematisch aus der Zeichnung ersichtlich ist. In der Zeichnung zeigt:

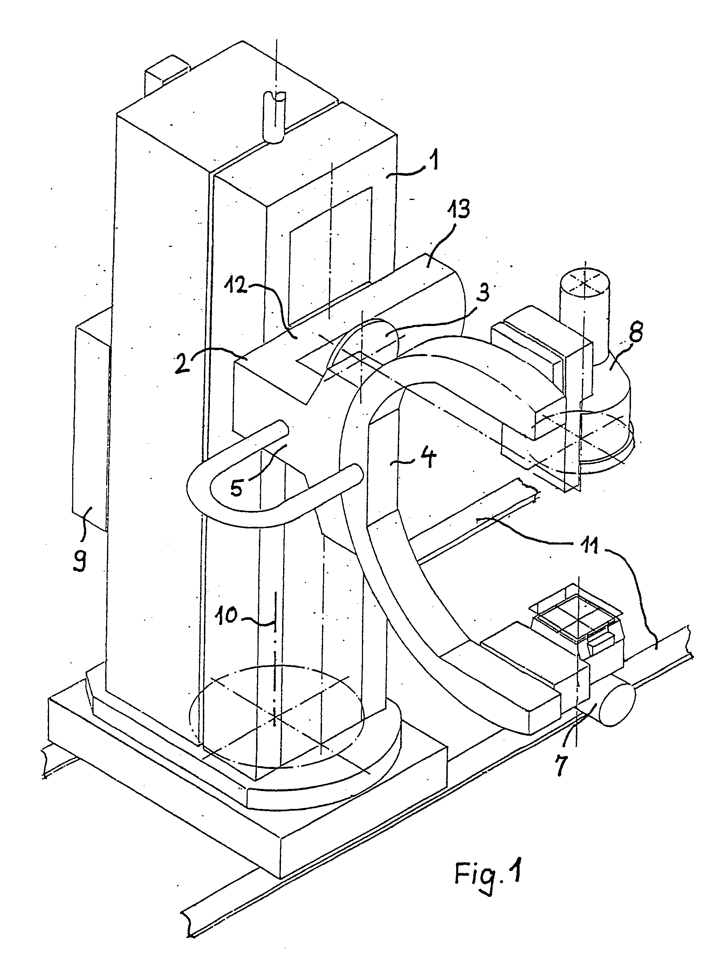

Fig. 1 eine perspektivische Ansicht des Angiographie-Gerätes schräg von vorne und

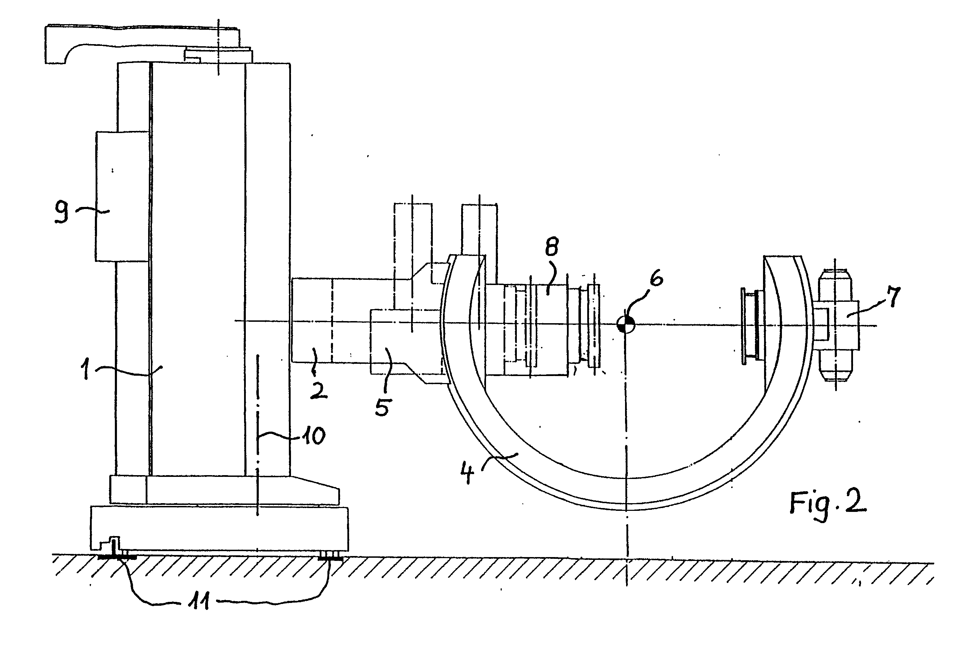

Fig. 2 eine Seitenansicht des Gerätes aus Figur 1 mit auf die horizontale Aufnahmerichtung eingestelltem C-Bogen.

Das erfindungsgemäße Angiographie-Gerät weist einen deckenfrei abgestützten Stand 1 auf, an dessen Vorderseite ein L-Arm 2 an einer horizontalen Welle 3 schwenkbar angeordnet ist, an dessen einem, nach vorn ragenden Schenkel 5 ein C-Bogen 4 um dessen Krümmungsmittelpunkt 6 drehverstellbar angebracht ist, im Bereich von dessen freien Enden eine Strahlereinheit 7 bzw. eine Bildverstärkereinheit 8 seitlich an der der Schwenkwelle 3 zugewandten Seite befestigt sind. Die Strahlereinheit 7 und die Bildverstärkereinheit 8 sind seitlich des C-Bogens 4 um einen solchen Abstand gegenüber diesem versetzt, daß die Strahlachse der Strahlereinheit 7 sich mit der Verlängerung der Achse der Welle 3 schneidet. Die Bildverstärkereinheit 8 ist außerdem in Strahlungsrichtung verstellbar. Auch die Strahlereinheit 7 kann in Strahlungsrichtung verstellbar sein.

Die horizontale Schwenkwelle 3 des L-Armes 2 ist in dem Stand 1 höhenverstellbar. Zur Entlastung des Höhenverstellantriebes ist an der Rückseite des Standes 1 ein Gegengewicht 9 angeordnet, das mit der Schwenkwelle 3 gegensinnig zu dieser höhenverstellbar gekuppelt ist. Von dem Gegengewicht 9 wird auch das Kippmoment aus dem Gewicht des auskragenden C-Bogens 4 und der Strahlereinheit 7 und Bildverstärkereinheit 8 wenigstens weitestgehend kompensiert.

Um auch das seitliche Kippmoment aus dem Gewicht des C-Bogens 4 und der daran sitzenden Einheiten 7, 8 um die Achse der Schwenkwelle 3 zu kompensieren und den Antrieb der Schwenkwelle 3 zu entlasten, ist der an dieser befestigte Schenkel 12 unter Ausbildung eines zweiten Gegengewichtes 13 über die Schwenkwelle 3 hinaus verlängert.

Der Stand 1 ist ferner um eine vertikale Achse 10 zu beiden Seiten hin um 90° drehbar, wobei die Achse 10 durch den Geräteschwerpunkt oder nahe an diesem verläuft. Außerdem ist der Stand 1 auf Schienen 11 zu beiden Seiten hin seitlich verfahrbar. Hierbei ist wenigstens die dem C-bogen 4 benachbarte vordere Schiene 11 in den Fußboden soweit eingelassen, daß sie mit ihrer Oberseite mit dem Bodenniveau abschließt und dadurch keine Stolperschwelle für die Bedienungspersonen bildet.

## Ansprüche

1. Angiographie-Gerät mit einer Strahlereinheit (7) und einer Bildverstärkereinheit (8), die im Bereich der Enden eines C-Bogens (4) angebracht sind, welcher um seinen Krümmungsmittelpunkt drehverstellbar an einem L-Arm (2) befestigt ist, der an einer horizontalen Welle (3) schwenkverstellbar an der Vorderseite eines deckenfreien Standes (1) angebracht ist, dadurch gekennzeichnet, daß die horizontale Schwenkwelle (3) des L-Arms (2) höhenverstellbar ist und daß mit der Schwenkwelle (3) ein mit dieser gegensinnig verstellbares, an der Rückseite des Standes (1) angeordnetes Gegengewicht (9) gekuppelt ist.

2. Angiographie-Gerät nach Anspruch 1, dadurch gekennzeichnet, daß der Stand (1) um eine vertikale Achse (10) drehbar ist, die durch den Geräteschwerpunkt oder nahe an diesem verläuft.

3. Angiographie-Gerät nach Anspruch 1 oder 2, dadurch gekennzeichnet, daß der Stand (1) auf Schienen (11) seitlich verfahrbar ist.

4. Angiographie-Gerät nach einem der Ansprüche 1 bis 3, dadurch gekennzeichnet, daß der an der horizontalen Schwenkwelle (3) befestigte Schenkel (12) des L-Arms (2) unter Ausbildung eines zweiten Gegengewichtes (13) über die Schwenkwelle (3) hinaus verlängert ist.

5. Angiographie-Gerät nach einem der Ansprüche 1 bis 4, dadurch gekennzeichnet, daß die Bildverstärkereinheit (8) und die Strahlereinheit (7) am C-Bogen (4) seitlich befestigt sind.

6. Angiographie-Gerät nach einem der Ansprüche 1 bis 5, dadurch gekennzeichnet, daß das Gerät einen Aufnahmepositionsspeicher aufweist, der von einem fahrbaren Bedienpult mit Aufnahmepositionstasten abrufbar ist.

7. Angiographie-Gerät nach einem der Ansprüche 1 bis 6, dadurch gekennzeichnet, daß das Gerät eine speicherprogrammierbare Steuerung aufweist.

8. Angiographie-Gerät nach einem der Ansprüche 1 bis 7, dadurch gekennzeichnet, daß das Gerät einen Infrarot-Fernbedienungseingang zur Ansteuerung der Antriebe der verstellbaren Geräte-

teile aufweist.

Fig.1

Fig. 2